(19) Europäisches Patentamt
European Patent Office
Office européen des brevets

(11) Veröffentlichungsnummer: **0 005 205**
**B1**

(12) **EUROPÄISCHE PATENTSCHRIFT**

(45) Veröffentlichungstag der Patentschrift: **09.09.81**

(21) Anmeldenummer: **79101155.4**

(22) Anmeldetag: **17.04.79**

(51) Int. Cl.³: **C 07 D 487/04,**
A 61 K 31/505
//(C07D487/04, 239/00,
209/00)

(54) **Substituierte 5,6-Dimethylpyrrolo(2,3-d)pyrimidine, Verfahren zu ihrer Herstellung und diese enthaltende Arzneimittel.**

(30) Priorität: **27.04.78 DE 2818676**

(43) Veröffentlichungstag der Anmeldung:
**14.11.79 Patentblatt 79/23**

(45) Bekanntmachung des Hinweises auf die Patenterteilung:
**09.09.81 Patentblatt 81/36**

(84) Benannte Vertragsstaaten:
**BE CH DE FR GB IT NL SE**

(56) Entgegenhaltungen:
**US - A - 3 037 980**
**US - A - 3 631 045**
**US - A - 3 867 386**
**US - A - 3 910 913**

**Chemical Abstracts, Band 83, Nr. 11**
**15. September 1975,**
**(COLUMBUS, OHIO, USA)**
**H. J. ROTH et al. "Synthesis of pyrrolo [2,3-d] pyrimidines"**
**Seite 577, Spalte 2, Abstract Nr. 97187r**

(73) Patentinhaber: **Troponwerke GmbH & Co. KG**
**Berliner Strasse 156**
**D-5000 Köln 80 (DE)**

(72) Erfinder: **Roth, Hermann J., Prof.Dr.**
**Böckingstrasse 4**
**D-5340 Bad Honnef (DE)**
Erfinder: **Eger, Kurt, Dr.**
**Dohmstrasse 2**
**D-5300 Bonn-Endenich (DE)**
Erfinder: **Issa, Sedika, Dr.**
**Bismarck Strasse 497 bei Frau Grothe**
**D-5090 Leverkusen 1 (DE)**
Erfinder: **Jacobi, Haireddin, Dr.**
**Finkenweg 2**
**D-5672 Leichlingen (DE)**

(74) Vertreter: **Dill, Erwin, Dr. et al,**
**c/o BAYER AG Zentralbereich Patente Marken und Lizenzen Bayerwerk**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 005 205 B1

Substituierte 5,6-Dimethylpyrrolo[2,3-d]pyrimidine, Verfahren zu ihrer Herstellung und diese enthaltende Arzneimittel

Die vorliegende Erfindung betrifft neue 5,6-Dimethylpyrrolo[2,3-d]pyrimidine, ein Verfahren zu ihrer Herstellung und diese enthaltende Arzneimittel, insbesondere als Antiphlogistika und als Mittel zur Behandlung des zentralen Nervensystems.

Einige Pyrrolo[2,3-d]pyrimidinderivate sind bereits als biologisch wirksame Verbindungen bekannt. So sind Derivate bekannt, die eine cytotoxische Aktivität entfalten (J. A. Montgomery et al., J.Med.Chem. *10* (1967), 665), von anderen ist eina antibiotische Wirksamkeit beschrieben (J. F. Gerster et al., J.Med.Chem. *10* (1967), 326). Die Herstellung ähnlicher Pyrrolo[2,3-d]pyrimidine ist ebenfalls beschrieben (H. J. Roth et al., Arch.Pharmaz., *308*, (1975), 252—258).

Diese Verbindungsklasse hat jedoch bisher noch keinen Eingang in die Humanmedizin gefunden.

Die Erfindung betrifft neue 5,6-Dimethylpyrrolo[2,3-d]pyrimidine der allgemeinen Formel (I)

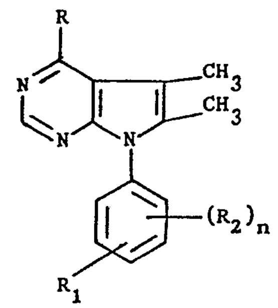

(I)

in welcher

R für eine Amino- oder Hydroxygruppe steht,

$R_1$ für ein Halogenatom, eine Nitrogruppe, eine Trifluormethylgruppe, eine Alkyl- oder Alkoxygruppe mit jeweils 1—2 Kohlenstoffatomen steht, und für den Fall, daß R eine Hydroxygruppe ist auch für Wasserstoff steht,

$R_2$ jeweils für ein Wasserstoffatom, ein Halogenatom, eine Methoxy- oder Methylgruppe steht, und

n für die Ziffern 1 oder 2 steht,

sowie ihre Säureadditionssalze.

Die neuen 5,6-Dimethylpyrrolo[2,3-d]pyrimidine der allgemeinen Formel (I), in welcher R, $R_1$, $R_2$ und n die oben angeführte Bedeutung haben, sind erhältlich, indem man Pyrrolderivate der allgemeinen Formel (II)

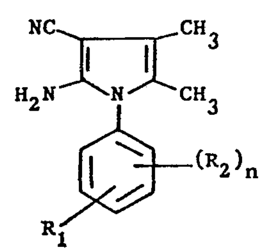

(II)

in welcher

$R_1$, $R_2$ und n die oben angeführte Bedeutung haben, mit Verbindungen der Formel (III)

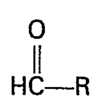

(III)

in welcher

R für die $NH_2$-Gruppe oder die Hydroxylgruppe steht, in Gegenwart von Ameisensäure und gegebenenfalls in Gegenwart von inerten organischen Lösungsmitteln bei erhöhter Temperatur umsetzt.

Für den Fall, daß nur Ameisensäure (R = OH) eingesetzt wird, erhält man Verbindungen der Formel (I), in denen R eine Hydroxylgruppe bedeutet und für den Fall, daß neben Ameisensäure ein Überschuß von Formamid eingesetzt wird, erhält man Verbindungen der Formel (I), in denen R eine Aminogruppe bedeutet.

Die erfindungsgemäßen Verbindungen der Formel (I) können mit geeigneten Säuren in physiologisch unbedenkliche Säureadditionssalze überführt werden. Diese Salze besitzen die gleichen vorteilhaften Eigenschaften wie die freien Verbindungen.

Die erfindungsgemäße Reaktion kann in Gegenwart oder Abwesenheit von Verdünnungsmitteln durchgeführt werden. Als Verdünnungsmittel kommen alle organischen Lösungsmittel in Frage, in welchen sich die Reaktionspartner lösen und die selbst nicht an der Reaktion teilnehmen. Besonders bewährt hat sich Dimethylformamid.

Die Reaktionstemperaturen können in einem größeren Bereich variiert werden. Im allgemeinen arbeitet man etwa zwischen 50 und 160°C, vorzugsweise zwischen 80 und 130°C.

Bei der Durchführung der erfindungsgemäßen Reaktionen setzt man pro Mol des jeweiligen Pyrrolderivates der allgemeinen Formel (II) einen Überschuß an Formamid und/oder Ameisensäure von 0,06 bis etwa 3 Mol, vorzugsweise von 1,5 bis 2,5 Mol, ein. Formamid und Ameisensäure können hier zugleich als Lösungsmittel dienen.

Die Aufarbeitung erfolgt in einfacher Weise durch Stehen der Reaktionslösung in der Kälte, Abfiltrieren des ausgefallenen Niederschlags und Rekristallisation aus einem geeigneten Lösungsmittel.

An neuen Wirkstoffen seien im einzelnen genannt:

5,6-Dimethyl-4-oxy-7-phenylpyrrolo[2,3-d]pyrimidin,
4-Amino-5,6-dimethyl-7-($\alpha,\alpha,\alpha$-trifluor-m-tolyl)pyrrolo[2,3-d]pyrimidin,
4-Amino-5,6-dimethyl-7-(4-fluorphenyl)pyrrolo[2,3-d]pyrimidin,
4-Amino-5,6-dimethyl-7-(2,4,5-trichlorphenyl)pyrrolo[2,3-d]pyrimidin,
4-Amino-5,6-dimethyl-7-(p-tolyl)pyrrolo[2,3-d]pyrimidin,
4-Amino-5,6-dimethyl-7-(o-tolyl)pyrrolo[2,3-d]pyrmidin,
4-Amino-5,6-dimethyl-7-(4-chlorphenyl)pyrrolo[2,3-d]pyrimidin,
4-Amino-5,6-dimethyl-7-(4-methoxyphenyl)pyrrolo[2,3-d]pyrimidin,
4-Amino-5,6-dimethyl-7-(4-methoxyphenyl)pyrrolo[2,3-d]pyrimidin,
4-Amino-5,6-dimethyl-7-(4-bromphenyl)pyrrolo[2,3-d]pyrimidin,
4-Amino-5,6-dimethyl-7-(2-bromphenyl)pyrrolo[2,3-d]pyrimidin,
4-Amino-5,6-dimethyl-7-(4-nitrophenyl)pyrrolo[2,3-d]pyrimidin,
4-Amino-5,6-dimethyl-7-(3,4-dichlorphenyl)pyrrolo[2,3-d]pyrimidin,
4-Amino-5,6-dimethyl-7-(3-chlorphenyl)pyrrolo[2,3-d]pyrimidin.

Die als Ausgangsverbindungen eingesetzten Pyrrolderivate der allgemeinen Formel (II) sind überwiegend neu, lassen sich aber aus Acetoin, dem entsprechenden Amin und Malonsäuredinitril nach folgendem Reaktionsschema herstellen:

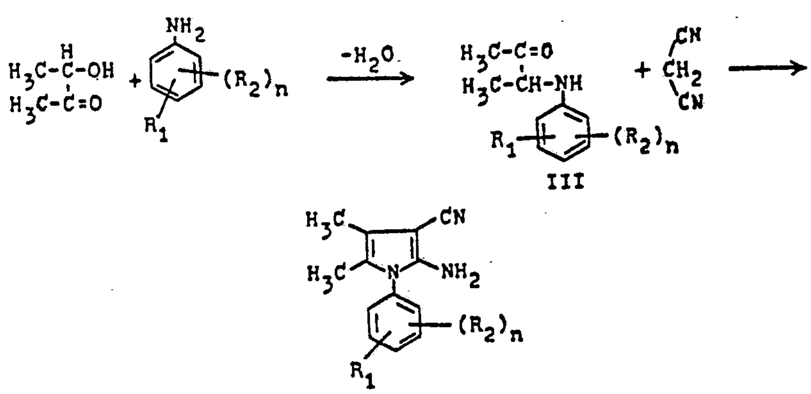

III

II

Bei dieser Darstellung erfolgt zunächst die Kondensation von Acetoin mit dem entsprechenden Amin in Gegenwart von katalytischen Mengen einer starken Säure wie z.B. Salzsäure oder p-Toluolsulfonsäure, in der Siedehitze in einem geeigneten Lösungsmittel, wie z.B. Benzol, unter Wasserabscheidung zu den $\alpha$-Aminoketonen der allgemeinen Formel (III), die jedoch nicht isoliert werden, sondern sofort mit Malonsäuredinitril erneut in der Hitze kondensiert werden.

Die Aufarbeitung erfolgt dann allgemein durch Abdampfen des Lösungsmittels und Rekristallisation des Rückstandes in einem geeigneten Lösungsmittel.

Im einzelnen seien die folgenden 1-substituierten 2-Amino-3-cyano-4,5-dimethylpyrrole genannt:

2-Amino-1-(3-chlorphenyl)-3-cyano-4,5-dimethylpyrrol,
2-Amino-1-(3,4-dichlorphenyl)-3-cyano-4,5-dimethylpyrrol,
2-Amino-1-(2-bromphenyl)-3-cyano-4,5-dimethylpyrrol,
2-Amino-1-(2-methoxyphenyl)-3-cyano-4,5-dimethylpyrrol,
2-Amino-1-(2-tolyl)-3-cyano-4,5-dimethylpyrrol,
2-Amino-1-(4-tolyl)-3-cyano-4,5-dimethylpyrrol,
2-Amino-1-(2,4,5-trichlorphenyl)-3-cyano-4,5-dimethylpyrrol,
2-Amino-1-(4-fluorphenyl)-3-cyano-4,5-dimethylpyrrol und
2-Amino-1-(3-trifluormethylphenyl)-2-amino-3-cyano-4,5-dimethylpyrrol.

Die erfindungsgemäßen Verbindungen weisen überraschenderweise eine Reihe vorteilhafter pharmakologischer Eigenschaften auf.

So zeigt ein Teil der Verbindungen nach oraler Applikation im hot-plate-Test einen deutlichen analgetischen Effekt, welcher stärker ist als der von Codein oder Dextropropoxyphen.

Wie am Modell der Balancestange ermittelt werden konnte, besitzt ein Teil der Verbindungen eine sedative Wirkungskomponente.

An den Modellen des Elektroschocks, des Pentetrazolschocks und des Nicotinkrampfes konnte gezeigt werden, daß ein Teil der erfindungsgemäßen Verbindungen eine deutliche antikonvulsive Wirksamkeit aufweisen. Bei einigen Verbindungen konnte im Traktionstest und im Ptosis-Test eine muskelrelaxierende Wirkung ermittelt werden.

Schließlich zeigten einige Verbindungen am Modell des Kaolinödems der Rattenpfote eine überraschend starke antiphlogistische Wirksamkeit.

Bei der Ermittlung der antiphlogistischen Wirkung bei einer repräsentativen Anzahl der erfindungsgemäßen Verbindungen zeigt sich, daß diese Verbindungen sowohl nach oraler als auch nach intramuskulärer Applikation eine unerwartet starke entzündungshemmende Wirkung besitzen. Die Testergebnisse sind aus der folgenden Tabelle zu ersehen. Die Wirkung wurde bestimmt am Kaolinödem der Rattenpfote (Methode: H. Jacobi et al., Arzneimittelforschung *27*, 1326, (1977)).

TABELLE

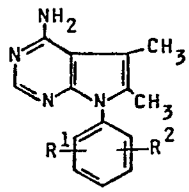

| Verbindung | $R^1$ | $R^2$ | Ödemhemmung in % der Kontrolle nach p.o. Applikation Dosis 100 mg/kg | Bemerkungen |
|---|---|---|---|---|
| A | (p) Cl | H | 85 | Ex. 1 |
| B | (p) $OCH_3$ | H | 82 | Ex. 3 |
| C | (p) Br | H | 71 | Ex. 4 |
| D | (o) Br | H | 74 | Ex. 5 |
| E | (o) $OCH_3$ | H | 73 | Ex. 8 |
| F | (o) $CH_3$ | H | 62 | Ex. 7 |
| G | (m) Cl | H | 57 | Ex. 13 |

Aufgrund dieser unerwarteten und vielseitigen Wirkungen stellen die erfindungsgemäßen Verbindungen eine Bereicherung der Pharmazie dar.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie Füll- und Streckmittel (z.B. Stärken, Milchzucker), Bindemittel (z.B.

Alginate, Gelatine, Polyvinylpyrrolidon), Feuchthaltemittel (z.B. Glycerin), Sprengmittel (z.B. Calciumcarbonat und Natriumbicarbonat), Netzmittel (z.B. Cetylalkohol, Glycerinmonostearat), Adsorptionsmittel (z.B. Kaolin und Bentonit) und Gleitmittel (z.B. Talkum, Calcium- und Magnesiumstearat), oder Gemische der aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben.

Suppositorien können neben dem oder den Wirkstoffen die üblichen wasserlöslichen oder wasserunlöslichen Trägerstoffe enthalten, z.B. Polyäthylenglykole, Fette und höhere Ester oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Traganth, Cellulosederivate, Polyäthylenglykole, Silicone, Bentonite, Talkum oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Alkohole, Äthylcarbonat, Propylenglykol, 1,3-Butylenglykol Öle, Glycerin, Polyäthylenglykole und Fettsäureester oder Gemische dieser Stoffe enthalten.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Traganth oder Gemische dieser Stoffe enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% oder Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral und rectal, appliziert werden.

## Beispiel 1

4-Amino-7-(4-chlorphenyl)-5,6-dimethylpyrrolo[2,3-d]pyrimidin

15 g (0,06 Mol) 2-Amino-1-(4-chlorphenyl)-3-cyano-4,5-dimethylpyrrol werden in 100 ml Formamid, 30 ml Dimethylformamid und 15 ml konzentrierter Ameisensäure 7 Stunden am Rückfluß erhitzt. Die beim Erkalten ausfallenden Kristalle werden mit Wasser gewaschen und aus äthanolischer Kalilauge umkristallisiert. Schmelzpunkt 250°C, Ausbeute 13 g (79% der Theorie).

$C_{14}H_{13}ClN_4$ (272,5)

| | | | | |
|---|---|---|---|---|
| berechnet: | C 61,65%, | H 4,7%, | N 20,5%, | Cl 13,0%; |
| gefunden: | C 61,73%, | H 4,78%, | N 20,30%, | Cl 13,9%. |

## Beispiel 2

4-Amino-7-(4-nitrophenyl)-5,6-dimethylpyrrolo[2,3-d]pyrimidin

Die Verbindung entsteht analog der in Beispiel 1 beschriebenen aus 0,03 Mol 1-(4-Nitrophenyl)-2-amino-3-cyano-4,5-dimethylpyrrol nach 18-stündigem Kochen. Schmelzpunkt 310°C, (Dimethylformamid), Ausbeute 5,6 g (64% der Theorie).

$C_{14}H_{13}N_5O_2$ (283)

| | | | |
|---|---|---|---|
| berechnet: | C 59,36%, | H 4,59%, | N 24,73%; |
| gefunden: | C 59,47%, | H 4,84%, | N 24,58%. |

## Beispiel 3

4-Amino-7-(4-methoxyphenyl)-5,6-dimethylpyrrolo[2,3-d]-pyrimidin

Analog Beispiel 1 aus 4,82 g (0,02 Mol) 1-(4-Methoxyphenyl)-2-amino-3-cyano-4,5-dimethylpyrrol in 7-stündiger Reaktionszeit. Schmelzpunkt 225°C (äthanolische KOH), Ausbeute: 4,5 g (85% der Theorie).

$C_{15}H_{16}N_4O$ (268)

berechnet: C 67,10%, H 5,90%, N 20,80%;

gefunden: C 67,09%, H 5,11%, N 20,53%.

Beispiel 4

4-Amino-7-(4-bromphenyl)-5,6-dimethylpyrrolo[2,3-d]pyrimidin

Analog Beispiel 1 aus 26 g (0,09 Mol) 1-(4-Bromphenyl)-2-amino-3-cyano-3,4-dimethylpyrrol in 7-stündiger Reaktion. Schmelzpunkt 245 bis 252°C (äthanolische KOH), Ausbeute 25 g (82,7% der Theorie).

$C_{14}H_{13}BrN_4$ (315,9)

berechnet: C 53,1%, H 4,11%, N 17,72%, Br 25,29%;

gefunden: C 52,97%, H 4,15%, N 17,55%, Br 25,33%.

Beispiel 5

4-Amino-7-(2-bromphenyl)-5,6-dimethylpyrrolo[2,3-d]pyrimidin

Analog Beispiel 1 aus 1-(2-Bromphenyl)-2-amino-3-cyano-4,5-dimethylpyrrol in 7-stündiger Reaktion. Schmelzpunkt 233 bis 235°C (äthanolische KOH), Ausbeute 2,4 g (79% der Theorie).

$C_{14}H_{13}BrN_4$ (315,9)

berechnet: C 53,1%, H 4,11%, N 17,72%, Br 25,29%;

gefunden: C 52,88%, H 4,12%, N 17,46%, Br 25,54%.

Analog den vorstehend beschriebenen Beispielen 1 bis 5 wurden hergestellt:

6) 4-Amino-7-(4-tolyl)-5,6-dimethylpyrrolo[2,3-d]pyrimidin
Schmelzpunkt 225°C.
$C_{15}H_{16}N_4$ (252)

berechnet: C 71,40%, H 6,39%, N 22,21%;

gefunden: C 71,37%, H 6,50%, N 22,13%.

7) 4-Amino-7-(2-tolyl)-5,6-dimethylpyrrolo[2,3-d]pyrimidin
Schmelzpunkt 195°C.
$C_{15}H_{14}N_4$ (252)

berechnet: C 71,40%, H 6,39%, N 22,21%;

gefunden: C 71,34%, H 6,58%, N 22,04%.

8) 4-Amino-7-(2-methoxyphenyl)-5,6-dimethylpyrrolo[2,3-d]pyrimidin
Schmelzpunkt 245°C.
$C_{15}H_{16}N_4O$ (268)

berechnet: C 67,5%, H 5,9%, N 20,8%;

gefunden: C 66,78%, H 5,96%, N 20,66%.

9) 4-Amino-7-(3-trifluoromethylphenyl)-5,6-dimethylpyrrolo[2,3-d]pyrimidin
Schmelzpunkt 230°C.
$C_{15}H_{13}F_3N_4$ (305)

berechnet: C 59,0%, H 4,26%, N 18,36%, F 16,27%;

gefunden: C 58,44%, H 4,30%, N 18,06%, F 18,66%.

10) 4-Amino-7-(4-fluorphenyl)-5,6-dimethylpyrrolo[2,3-d]-pyrimidin
Schmelzpunkt 232°C.
$C_{14}H_{13}FN_4$(256)

berechnet: C 65,62%, H 5,07%, N 21,8%, F 7,41%;

gefunden: C 65,54%, H 5,12%, N 21,4% F 7,3%.

11) 4-Amino-7-(2,4,5-trichlorphenyl)-5,6-dimethylpyrrolo[2,3-d]pyrimidin
Schmelzpunkt 248°C.
$C_{14}H_{11}Cl_3N_4$ (341,5)

berechnet: C 49,26%, H 3,25%, N 16,39%, Cl 31,18%;

gefunden: C 49,20%, H 3,30%, N 16,41%, Cl 31,09%.

12) 4-Amino-7-(3,4-dichlorphenyl)-5,6-dimethylpyrrolo[2,3-d]-pyrimidin
Schmelzpunkt 270°C.
$C_{14}H_{12}Cl_2N_4$

berechnet: C 54,72%, H 3,90%, N 18,2% Cl 23,15%;

gefunden: C 54,68%, H 4,06%, N 18,08%, Cl 23,18%.

13) 4-Amino-7-(3-chlorphenyl)-5,6-dimethylpyrrolo[2,3-d]-pyrimidin
Schmelzpunkt: 210°C.
$C_{14}H_{13}ClN_4$ (272,5)

berechnet: C 61,65%, H 4,7%, N 20,50%, Cl 13,0%;

gefunden: C 61,45%, H 4,9%, N 20,53%, Cl 13,12%.

Beispiel 14

5,6-Dimethyl-4-hydroxy-7-phenylpyrrolo[2,3-d]pyrimidin

6,2 g (0,03 Mol) 2-Amino-3-cyano-4,5-dimethyl-1-phenylpyrrol werden in 60 ml 85 %iger Ameisensäure 12 Stunden am Rückfluß gekocht. Die beim Erkalten ausfallenden Kristalle werden aus Dimethylformamid umkristallisiert.

Schmelzpunkt: 295°C, Ausbeute: 4,2 g (64% der Theorie).

Die als Ausgangsmaterial dienenden substituierten Pyrrole sind bisher in der Literatur zumeist nicht beschrieben worden. Sie lassen sich in einfacher Weise nach der folgenden allgemeinen Vorschrift herstellen:

Äquimolare Mengen Acetoin und entsprechendes Amin (Größenordnung 5 bis 15 g) werden in 80 bis 100 ml Benzol gelöst (erforderlichenfalls mit 3 Tropfen konzentrierter Salzsäure oder 0,1 g p-Toluolsulfonsäure versetzt) und im Wasserabscheider solange gekocht, bis die theoretische Menge Wasser abgeschieden worden ist. Zu dieser noch warmen Reaktionslösung werden äquimolare Mengen Malonitril (erforderlichenfalls wird etwas Piperidin zugesetzt) zugefügt und das Gemisch weiter bis zur erforderlich abzuscheidenden Wassermenge gekocht. Nach dem Abdampfen des Lösungsmittels im Vakuum wird der Rückstand aus Alkohol oder Dimethylformamid rekristallisiert.

Nach dieser Methode lassen sich die folgenden Pyrrole darstellen:

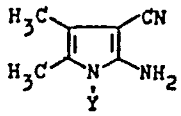

| Y | Schmelzpunkt | Ausbeute |
|---|---|---|
| 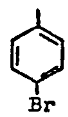 | 167° C | 89,6 % |
| 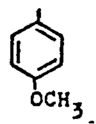 | 162–164° C | 49 % |
| 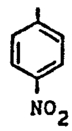 | 207° C | 74 % |
| 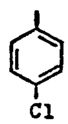 | 164° C | 73 % |
| 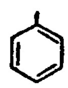 | 135° C | 65 % |
| 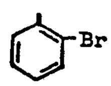 | 135° C | 21 % |
| 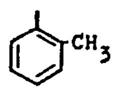 | 125° C | 36 % |
| 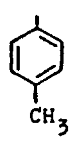 | 163–165° C | 34 % |
| 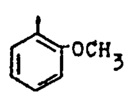 | 120° C | 30 % |
| 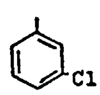 | 158° C | 21 % |
| 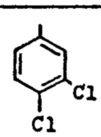 | 175° C | 22 % |

Fortsetzung:

| Y | Schmelzpunkt | Ausbeute |
|---|---|---|
| 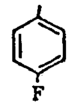 | 115° C | 22 % |
| 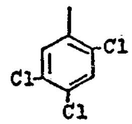 | 180° C | 25 % |

**Patentansprüche**

1. 5,6-Dimethylpyrrolo[2,3-d]pyrimidine der allgemeinen Formel (I)

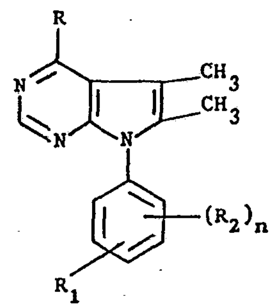

in welcher

R für eine Amino- oder Hydroxygruppe steht,

$R_1$ für ein Halogenatom, eine Nitrogruppe, eine Trifluormethylgruppe, eine Alkyl- oder Alkoxygruppe mit jeweils 1—2 Kohlenstoffatomen steht, und für den Fall, daß R eine Hydroxygruppe ist auch für Wasserstoff steht,

$R_2$ jeweils für ein Wasserstoffatom, ein Halogenatom, eine Methoxy- oder Methylgruppe steht, und

n für die Zifferns 1 oder 2 steht,

sowie ihre Säureadditionssalze.

2. Verbindungen gemäß Anspruch 1, dadurch gekennzeichnet, daß R für eine Aminogruppe steht.

3. Verfahren zur Herstellung von 5,6-Dimethylpyrrolo[2,3-d]-pyrimidinen der Formel (I) gemäß Anspruch 1, dadurch gekennzeichnet, daß man Pyrrolderivate der allgemeinen Formel (II)

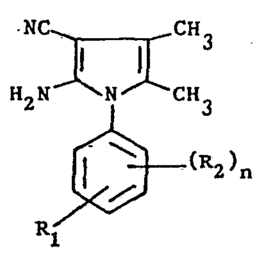

in welcher

$R_1$, $R_2$ und n die oben angeführte Bedeutung haben, mit Verbindungen der Formel (III)

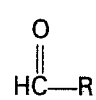 (III)

in welcher

R für die $NH_2$-Gruppe oder die Hydroxylgruppe steht, in Gegenwart von Ameisensäure und gegebenenfalls in Gegenwart von inerten organischen Lösungsmitteln bei erhöhter Temperatur umsetzt.

4. Verfahren zur Herstellung von Verbindungen der Formel (I) in welcher R für eine Hydroxygruppe steht gemäß Anspruch 3, dadurch gekennzeichnet, daß man Pyrrolderivate der Formel (II) mit einem Überschuß von Ameisensäure umsetzt.

5. Verfahren gemäß Anspruch 3, dadurch gekennzeichnet, daß man einen Überschuß von Formamid einsetzt.

6. Arzneimittel enthaltend 5,6-Dimethylpyrrolo[2,3-d]-pyrimidine gemäß Formel (I) in Anspruch 1.

## Claims

1. 5,6-dimethylpyrrolo[2,3-d]pyrimidines of the general formula (I)

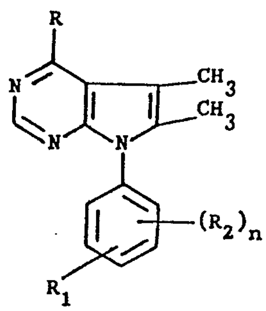

in which

R represents an amino or hydroxyl group,

$R_1$ represents a halogen atom, a nitro group, a trifluoromethyl group, an alkyl or alkoxy group with in each case 1—2 carbon atoms and in the case where R is a hydroxyl group, also represents hydrogen,

$R_2$ in each case denotes a hydrogen atom, a halogen atom, a methoxy or methyl group,

and

n represents the figures 1 or 2, and

acid addition salts thereof.

2. Compounds according to claim 1, characterized in that R represents an amino group.

3. Process for the preparation of 5,6-dimethylpyrrolo[2,3-d]pyrimidines of the formula (I) according to claim 1, characterised in that pyrrole derivatives of the general formula (II)

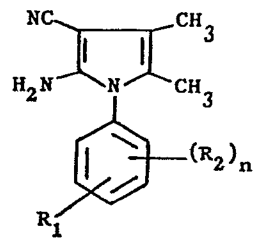

in which

$R_1$, $R_2$ and n have the meaning given above, are reacted with compounds of the formula (III)

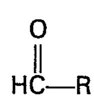

(III)

in which

R represents the $NH_2$ group or the hydroxyl group, in the presence of formic acid and optionally in the presence of inert organic solvents at elevated temperature.

4. Process for the preparation of compounds of formula (I) in which R represents a hydroxyl group, according to claim 3, characterised in that pyrrole derivatives of the formula (II) are reacted with an excess of formic acid.

5. Process according to claim 3, characterised in that an excess of formamide is used.

6. Medicaments containing 5,6-dimethylpyrrolo[2,3-d]-pyrimidines according to formula (I) in claim 1.

**Revendications**

1. 5,6-diméthylpyrrolo[2,3-d]pyrimidines de formule générale (I):

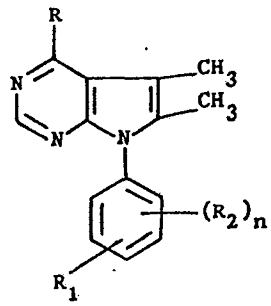

dans laquelle
R représente un groupe amino ou un groupe hydroxy,
$R_1$ représente un atome d'halogène, un groupe nitro, un groupe trifluorométhyle, un groupe alkyle ou un groupe alcoxy contenant chacun 1 ou 2 atomes de carbone et, si R est un groupe hydroxy, $R_1$ représente également un atome d'hydrogène,
chaque radical $R_2$ représente un atome d'hydrogène, un atome d'halogène, un groupe méthoxy ou un groupe méthyle, et
n représente les chiffres 1 ou 2,
ainsi que leurs sels d'addition d'acide.

2. Composés suivant la revendication 1, caractérisé en ce que R représente un groupe amino.

3. Procédé de préparation de 5,6-diméthylpyrrolo[2,3-d]pyrimidines de formule (I) suivant la revendication 1, caractérisé en ce qu'on fait réagir des dérivés de pyrrole de formule générale (II):

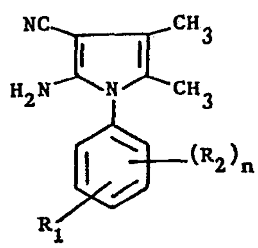

dans laquelle
$R_1$, $R_2$ et n ont les significations indiquées ci-dessus,
avec des composés de formule (III):

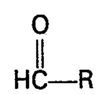

(III)

dans laquelle
R représente le groupe $NH_2$ ou le groupe hydroxy, en présence d'acide formique et éventuellement en présence de solvants organiques inertes à température élevée.

4. Procédé de préparation de composés de formule (I) dans laquelle R représente un groupe hydroxy, suivant la revendication 3, caractérisé en ce qu'on fait réagir des dérivés de pyrrole de formule (II) avec un excès d'acide formique.

5. Procédé suivant la revendication 3, caractérisé en ce qu'on utilise un excès de formamide.

6. Médicaments contenant des 5,6-diméthylpyrrolo[2,3-d]pyrimidines répondant à la formule (I) suivant la revendication 1.